(19) Europäisches Patentamt / European Patent Office / Office européen des brevets

(11) **EP 4 722 724 A1**

(12) **EUROPEAN PATENT APPLICATION**
published in accordance with Art. 153(4) EPC

(43) Date of publication:
08.04.2026  Bulletin 2026/15

(21) Application number: 24815501.2

(22) Date of filing: 29.05.2024

(51) International Patent Classification (IPC):
*G01N 33/86* (2006.01)

(52) Cooperative Patent Classification (CPC):
G01N 33/86

(86) International application number:
PCT/JP2024/019652

(87) International publication number:
WO 2024/248019 (05.12.2024 Gazette 2024/49)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 30.05.2023  JP 2023089032

(71) Applicant: Sekisui Medical Co., Ltd.
Tokyo 103-0027 (JP)

(72) Inventors:
• ODA, Yukio
  Tokyo 103-0027 (JP)
• EMMI, Mari
  Tokyo 103-0027 (JP)

(74) Representative: Wächtershäuser & Hartz
Patentanwaltspartnerschaft mbB
Weinstraße 8
80333 München (DE)

(54) **METHOD FOR ESTIMATING CAUSE OF BLOOD COAGULATION ANOMALY BY ANALYSIS OF DEVIATION WAVEFORM**

(57)  A method for estimating a cause for coagulation disorder in a blood specimen, the method comprising: calculating D(i), which is a deviation curve for a subject blood specimen; calculating a parameter reflecting a shape of D(i); and estimating a cause for coagulation disorder in the subject blood specimen on a basis of the parameter, wherein D(i) - P(i) - Pn(i), wherein i denotes measuring point number, or time, P(i) is a curve given by relativizing measurement data for coagulation reaction in the subject blood specimen to give a minimum value of 0 and a maximum value of A, Pn(i) is a curve given by shifting P'(i) to match a rising point thereof with a specific point X, wherein the P'(i) is a curve given by relativizing measurement data for coagulation reaction in a reference specimen to give a minimum value of 0 and a maximum value of A, A > 0, and X is from 5 to 15 seconds, or measuring point number corresponding thereto.

Fig. 2

(Cont. next page)

Fig. 2

# Fig. 2

**Description**

Technical Field

The present invention relates to a method for estimating a cause for blood coagulation disorder.

Background Art

Blood coagulation test is a test to diagnose the blood coagulability of a patient by measuring blood coagulation time or the like with addition of a specific reagent to a blood specimen from the patient. Typical examples of blood coagulation time include prothrombin time (PT), activated partial thromboplastin time (APTT), and thrombin time. Abnormality in blood coagulability results in extension of coagulation time. Causes for the occurrence of abnormality in blood coagulability include the influence of an anticoagulant drug, reduction of components involved in coagulation, congenital deficiency of blood coagulation factors, and acquired development of autoantibodies which inhibit coagulation reaction.

When extension of coagulation time is found for a subject specimen in the conventional blood coagulation test, cross-mixing test is typically carried out to determine the cause for extended coagulation time if the specimen is undoubtedly free of any anticoagulant drug. In the cross-mixing test, a mixed specimen of the subject specimen and a normal specimen is prepared, APTT immediately after mixing (immediate reaction) and APTT after incubation at 37°C for 2 hours (delayed reaction) are measured, and which of a coagulation factor inhibitor (anticoagulant), lupus anticoagulant (LA), and coagulation factor deficiency such as hemophilia is the cause for the extension of APTT in the subject specimen is determined on the basis of the patterns of the immediate reaction and delayed reaction. However, the conventional cross-mixing test requires very much time and effort as mentioned.

Directly acting oral anticoagulants (DOACs) are oral anticoagulant drugs which inhibit thrombin or activated factor X, and prophylactically administered to patients having the risk of thrombosis. Blood specimens collected from patients who have received DOACs administration normally exhibit extended coagulation time, but in some cases exhibit coagulation time within the normal range of time; this may cause a serious danger to patients. For example, suppose that a patient who has received DOACs administration is emergency-transported and receives surgery because of a traffic accident or the like; if extension of coagulation time is not observed in the common presurgical coagulation test, physicians who do not know that the patient has received DOACs administration cannot notice the presence of abnormality in the coagulability of the patient, failing to properly treat the patient.

Patent Literatures 1 to 4 describe calculation of parameters including weighted average time, weighted average height, peak width, and baseline length in a coagulation rate curve for a blood specimen, and use of these parameters for estimation of a cause for extension of blood coagulation time, evaluation of coagulation factor deficiency, calculation of blood coagulation time, and so on. Patent Literature 5 describes calculation of parameters for estimation of a cause for blood coagulation disorder by using $p_k$ and $q_k$, wherein $p_k$ and $q_k$ are calculated as points before and after a coagulation rate curve reaches a maximum value at each of which the coagulation rate curve reaches S% of a maximum value thereof. Patent Literature 6 describes estimation of a cause for extension of blood coagulation time with reference to the shape of a function, T(X), representing time at which a coagulation reaction curve, P(i), for a blood specimen reaches X% of a coagulation reaction end point, Pe.

Citation List

Patent Literature

Patent Literature 1: WO 2020/101025
Patent Literature 2: WO 2020/158948
Patent Literature 3: WO 2020/218425
Patent Literature 4: WO 2021/177452
Patent Literature 5: WO 2022/054819
Patent Literature 6: WO 2022/186381

Summary of Invention

Technical Problem

The present invention provides a novel method for estimating a cause for coagulation disorder in a blood specimen.

Solution to Problem

Specifically, the present invention provides the followings.

[1] A method for estimating a cause for coagulation disorder in a blood specimen, the method comprising:

calculating D(i), which is a deviation curve for a subject blood specimen;
calculating a parameter reflecting a shape of D(i); and
estimating a cause for coagulation disorder in the subject blood specimen on a basis of the parameter, wherein

$$D(i) = P(i) - Pn(i),$$

wherein
i denotes measuring point number, or time,
P(i) is a curve given by relativizing measurement data for coagulation reaction in the subject blood specimen to give a minimum value of 0 and a maximum value of A,
Pn(i) is a curve given by shifting P'(i) to match a rising point thereof with a specific point X, wherein the P'(i) is a curve given by relativizing measurement data for coagulation reaction in a reference specimen to give a minimum value of 0 and a maximum value of A,

$$A > 0,$$

and
X is from 5 to 15 seconds, or measuring point number corresponding thereto.

[2] The method according to [1], wherein the parameter reflecting the shape of D(i) is at least one selected from the group consisting of gT(S), gH(S), gB(S), gAB(S), and RgB(S1/S2), wherein

gT(S) and gH(S) are expressed as the following equations:

$$gT(S) = \frac{\sum_{i=t1}^{t2}(i \times D(i))}{\sum_{i=t1}^{t2} D(i)}$$

$$gH(S) = \frac{\sum_{i=t1}^{t2}(i \times D(i))}{\sum_{i=t1}^{t2} i}$$

wherein
t1 and t2 are respectively a minimum value and maximum value of i satisfying $D(i) \geq Dmax \times S(\%)$, wherein Dmax denotes a maximum value of D(i), and $0 < S < 100$,
provided that if $D(i) < Dmax \times S(\%)$, then i = 0 and D(i) = 0,
gB(S) represents time length or the number of measuring points satisfying $D(i) \geq Dmax \times S(\%)$ between t1 and t2,

$$gAB(S) = gH(S) / gB(S),$$

and

$$RgB(S1/S2) = gB(S1) / gB(S2),$$

wherein
$0 < S1 < 100$ and $0 < S2 < 100$, provided that $S1 \neq S2$.

[3] The method according to [2], comprising calculating at least one selected from the group consisting of gB(S) and gAB(S) if extension of coagulation time is absent in the subject blood specimen.

[4] The method according to [3], comprising estimating the subject blood specimen to be DOACs-positive if gB(S) is equal to or greater than a threshold or gAB(S) is equal to or smaller than a threshold.

[5] The method according to [2], comprising calculating at least one selected from the group consisting of gAB(S) and RgB(S1/S2) if extension of coagulation time is present in the subject blood specimen.

[6] The method according to [5], comprising estimating the subject blood specimen to be coagulation-factor-inhibitor-positive if gAB(S) is equal to or smaller than a threshold.

[7] The method according to [5], comprising estimating the subject blood specimen to be heparin-positive if S1 < S2, and RgB(S1/S2) is equal to or smaller than a threshold, or if S1 > S2, and RgB(S1/S2) is equal to or greater than a threshold.

[8] The method according to [5], comprising:

estimating the subject blood specimen to be coagulation-factor-inhibitor-positive if gAB(S) is equal to or smaller than a threshold;
estimating the subject blood specimen to be heparin-positive if S1 < S2, and RgB(S1/S2) is equal to or smaller than a threshold, or if S1 > S2, and RgB(S1/S2) is equal to or greater than a threshold; and
estimating whether a subject blood specimen estimated not to be coagulation-factor-inhibitor-positive or heparin-positive is lupus-anticoagulant-positive or coagulation-factor-deficient.

[9] The method according to [8], comprising:

preparing a mixed specimen of the subject blood specimen estimated not to be coagulation-factor-inhibitor-positive or heparin-positive and a normal specimen;
calculating Dm(i), which is a deviation curve for the mixed specimen, and Ds(i), which is a deviation curve for the normal specimen;
calculating gAB(S) for each of Dm(i) and Ds(i); and
calculating a parameter R(S), wherein

$$Dm(i) = Pm(i) - Pn(i),$$

$$Ds(i) = Ps(i) - Pn(i),$$

wherein
Pm(i) is a curve given by relativizing measurement data for coagulation reaction in the mixed specimen to give a minimum value of 0 and a maximum value of A,
Ps(i) is a curve given by relativizing measurement data for coagulation reaction in the normal specimen to give a minimum value of 0 and a maximum value of A, and Pn(i) and A are as defined above,
gAB(S) for Ds(i) is expressed as the following equation:

gAB(S) for Ds(i) = [gH(S) for Ds(i)] / [gB(S) for Ds(i)]

wherein

$$gH(S) \ for \ Ds(i) = \frac{\sum_{i=t1}^{t2}(i \times Ds(i))}{\sum_{i=t1}^{t2}i} \qquad (I)$$

wherein
t1 and t2 are respectively a minimum value and maximum value of i satisfying Ds(i) ≥ [maximum value of Ds(i) × S(%)],

$$0 < S < 100,$$

provided that if Ds(i) < [maximum value of Ds(i) × S(%)], then i = 0 and Ds(i) = 0 are assigned, and
gB(S) for Ds(i) represents time length or the number of measuring points satisfying Ds(i) ≥ [maximum value of Ds(i) × S(%)] between t1 and t2,
gAB(S) for Dm(i) is expressed as the following equation:

gAB(S) for Dm(i) = [gH(S) for Dm(i)] / [gB(S) for Dm(i)]

wherein

$$gH(S) \ for \ Dm(i) = \frac{\sum_{i=t1}^{t2}(i \times Dm(i))}{\sum_{i=t1}^{t2} i} \qquad (II)$$

wherein
t1 and t2 are respectively a minimum value and maximum value of i satisfying Dm(i) ≥ [maximum value of Dm(i) × S(%)],

$$0 < S < 100,$$

provided that if Dm(i) < [maximum value of Dm(i) × S(%)], then i = 0 and Dm(i) = 0 are assigned, and gB(S) for Dm(i) represents time length or the number of measuring points satisfying Dm(i) ≥ [maximum value of Dm(i) × S(%)] between t1 and t2, and R(S) = {(gAB(S) for Ds(i)) + (gAB(S) for D(i))} / 2 - (gAB(S) for Dm(i)).

[10] The method according to [9], comprising estimating the subject blood specimen to be lupus-anticoagulant-positive if R(S) is equal to or greater than a threshold, or estimating the subject blood specimen to be coagulation-factor-deficient if R(S) is equal to or smaller than a threshold.
[11] The method according to [1], further comprising calculating coagulation time for the subject blood specimen.

Advantageous Effects of Invention

According to the present invention, when extension of coagulation time is present in a subject blood specimen, the cause for coagulation disorder which has resulted in the extension of coagulation time can be estimated. According to the present invention, a blood specimen which has received administration of DOACs can be detected from subject blood specimens in which extension of coagulation time is not found and coagulation disorder cannot be detected in conventional coagulation test.

Brief Description of Drawings

Fig. 1 shows P(i) and Pn(i) for a reference specimen (RP).
Fig. 2 shows P(i) and D(i) for a coagulation-factor-inhibitor-positive (Inh) specimen (A), a heparin-positive (Hep) specimen (B), and a DOACs-positive (DOACs) specimens (C); left: P(i) and Pn(i), right: D(i).
Fig. 3 shows gB50(A), gB60(B), gB70(C), and gAB60(D) for DOACs-positive (DOACs) specimens and other APTT-normal specimens.
Fig. 4 shows gAB05 for coagulation-factor-inhibitor-positive (Inh) specimens and specimens with any other cause for APTT extension.
Fig. 5 shows gB05 / gB90 for heparin-positive (Hep) specimens and specimens with any other cause for APTT extension.
Fig. 6 shows R(S) (S = 5, 10, 20, 30, 40, and 50) for mixed specimens derived from LA-positive (LA) specimens and those derived from coagulation-factor-deficient (Def) specimens.
Fig. 7 shows R(S) (S = 60, 70, 80, and 90) for mixed specimens derived from LA-positive (LA) specimens and those derived from coagulation-factor-deficient (Def) specimens.

Description of Embodiments

In blood coagulation test, a specific reagent is added to a subject blood specimen, and the subsequent blood coagulation reaction is monitored to measure the blood coagulation time from the coagulation reaction. Herein, the terms blood coagulation reaction, blood coagulation time, and blood specimen may be simply referred to as "coagulation reaction", "coagulation time", and "specimen", respectively. For monitoring of coagulation reaction, common means are used, such as optical means to measure scattered light intensity, transmittance, absorbance, or the like, or mechanical means to measure the viscosity of plasma. Although varying among different measuring means, coagulation reaction curves for normal specimens basically exhibit sigmoidal shapes. For example, coagulation reaction curves based on scattered light intensity for normal specimens normally exhibit rapid elevation due to the progress of coagulation after some time has passed from addition of a reagent, and thereafter a plateau is reached as the coagulation reaction approaches the end. On the other hand, coagulation reaction curves for abnormal specimens with abnormality in blood coagulation exhibit various shapes depending on causes for abnormality, such as delay of rising or slow elevation of the curve. As a result, abnormal specimens often exhibit longer coagulation time than normal specimens. The extension of coagulation time is an indicator of the presence of a cause for coagulation disorder. However, it is impossible to estimate the cause type for coagulation disorder (the cause for extension of coagulation time) from coagulation time.

When extension of coagulation time such as APTT is found in blood coagulation test, contamination with heparin (for example, administration of heparin, blood collection from a heparin-locked intravenous infusion line, blood collection after dialysis) is first suspected to be the cause in typical cases. Blood specimens contaminated with heparin can be confirmed from reduction in coagulation time to be caused by protamine sulfate or the like, which has an effect to neutralize heparin. If a blood specimen is undoubtedly uncontaminated with heparin, the presence or absence of administration of DOACs or warfarin is checked if possible, and cross-mixing test is then carried out to determine the cause for extension of coagulation time (hereinafter, also referred to as "the cause for the extension", simply). Specifically, which of a coagulation factor inhibitor (anticoagulant), lupus anticoagulant (LA), and coagulation factor deficiency such as hemophilia is the cause for the extension of APTT is determined. In the cross-mixing test, APTT immediately after mixing (immediate reaction) and APTT after incubation at 37°C for 2 hours (delayed reaction) are measured for a normal specimen, a subject specimen, and a mixed specimen of the normal specimen and the subject specimen. The cause for the extension of APTT is determined on the basis of the patterns of these immediate reaction and delayed reaction. In the conventional method, cross-mixing test must be carried out separately from coagulation time measurement for successful determination of the cause for the extension. However, the cross-mixing test needs monitoring of immediate reaction and delayed reaction after incubation for 2 hours for a mixed specimen, thus requiring much time and effort.

As Patent Literatures 1 to 5 described above, methods for acquiring data on the cause for the extension without cross-mixing test have been proposed. The evaluation of the cause for the extension in those literatures uses differential curves of a coagulation reaction curve, specifically, the coagulation rate (first derivative) and coagulation acceleration (second derivative). In Patent Literature 6, not such differential curves but a function, $T(X)$, calculated on the basis of a coagulation reaction curve is used to extract information on the cause for the extension. However, almost no attempt has been made to extract information on the cause for the extension with a focus on coagulation reaction curves.

[Method for estimating cause for extension of coagulation time]

The present invention relates to a method for estimating a cause for coagulation disorder in a blood specimen. In the method of the present invention, the cause for coagulation disorder is estimated by analyzing the difference between a coagulation reaction curve for a subject specimen and that for a reference specimen.

1. Coagulation reaction monitoring

In coagulation reaction monitoring, coagulation reaction in the subject specimen is monitored. A coagulation reaction curve can be generated from time-series data acquired in this monitoring for coagulation reaction. The coagulation reaction monitoring can be carried out with a common coagulation reaction monitoring procedure, for example, to be performed in measurement of coagulation time such as prothrombin time (PT) and activated partial thromboplastin time (APTT) or measurement of fibrinogen concentration (Fbg). Hereinafter, the method of the present invention is described mainly on the basis of coagulation reaction monitoring by APTT measurement. Those skilled in the art could modify the method of the present invention by replacing with another monitoring method (for example, coagulation reaction monitoring by PT measurement).

In the coagulation reaction monitoring, the plasma of a test subject is preferably used as a subject blood specimen. To the specimen, an anticoagulant agent which is commonly used in coagulation test can be added. For example, blood is collected with a blood collection tube containing sodium citrate, and then centrifuged to give plasma.

In the coagulation reaction monitoring, a reagent for coagulation time measurement is added to the subject specimen to initiate blood coagulation reaction. The coagulation reaction in the mixed solution after adding the reagent can be monitored. Any reagent meeting the purpose of measurement can be selected for use. Reagents for various types of coagulation time measurement are commercially available (for example, the APTT reagent Coagpia APTT-N; manufactured by SEKISUI MEDICAL CO., LTD.). For monitoring of the coagulation reaction, a common means can be used, such as an optical means to measure scattered light intensity, transmittance, absorbance, or the like, or mechanical means to measure the viscosity of plasma. In typical cases, the starting time point of the coagulation reaction can be specified as the time point at which the reagent is mixed with the specimen to initiate coagulation reaction, but another time point may be specified as the starting time point of the reaction. The time period for monitoring the coagulation reaction can be arbitrarily set, and can be, for example, from several tens of seconds to about 7 minutes after the time point of mixing the specimen and the reagent. During the time period of monitoring, measurement can be repeatedly performed (photometry for optical detection) at specific intervals for the state of progress of the coagulation reaction. For example, measurement can be performed at intervals of 0.1 seconds. The mixed solution during the monitoring is under a normal temperature condition; for example, the temperature is 30°C or more and 40°C or less, and preferably 35°C or more and 39°C or less. Appropriate conditions for the monitoring can be set to fit with the subject specimen, the reagent, the monitoring means, and so on.

A series of operations in the above-described coagulation reaction monitoring can be performed with an autoanalyzer. An example of the autoanalyzer is the automatic blood coagulation analyzer CP3000 (manufactured by SEKISUI MEDICAL CO., LTD.). Some operations may be manually performed. For example, preparation of the subject specimen can be manually performed with the subsequent operations performed with an autoanalyzer.

2. Coagulation reaction curve

Through the coagulation reaction monitoring, measurement data for the subject specimen are acquired. These are data reflecting the process of the coagulation reaction in the subject specimen. For example, data showing the temporal variation of the degree of progress of the coagulation reaction (for example, scattered light intensity) after addition of calcium chloride solution (hereinafter, referred to as reaction data, $C(i)$) are acquired from the mixed solution containing the subject specimen and the reagent for coagulation time measurement.

The reaction data, $C(i)$, are preprocessed, as necessary. The preprocessing can include smoothing to remove noises or zero adjustment. For the smoothing, any known noise removal method can be used. The mixed solution containing the subject specimen exhibits a scattered light intensity of more than 0 at the starting time point of the reaction (time 0). Through the zero adjustment, the scattered light intensity at time 0 can be adjusted to 0. The zero adjustment is preferably performed after the smoothing.

Then, the reaction data, $C(i)$, are relativized to give a coagulation reaction curve, $P(i)$, to be used in the present invention. Preferably, $P(i)$ is determined from $C(i)$ with an equation (1) shown below. In the equation (1), Cmax and Cmin respectively denote the maximum value and minimum value of $C(i)$, Crange denotes the variation range of $C(i)$ (that is, Cmax - Cmin), and A is an arbitrary value and corresponds to the maximum value of $P(i)$. i denotes time after the start of the coagulation reaction in the specimen (hereinafter, also referred to as "time", simply) or the number of measuring points after the start of the coagulation reaction in the specimen (hereinafter, also referred to as "measuring point number", simply).

$$P(i) = [(C(i) - Cmin) / Crange] \times A \quad (1)$$

(A is arbitrary, provided that A > 0, for example, A = 100)

$P(i)$ based on scattered light intensity is normally sigmoidal. By contrast, $P(i)$ based on transmitted light intensity is normally reverse-sigmoidal. Hereinafter, the procedure of the method of the present invention is described with $P(i)$ based on scattered light intensity.

3. Deviation curve

In the present invention, a deviation curve for the subject specimen is generated from the coagulation reaction curve, $P(i)$. The deviation curve represents the difference between $P(i)$ for the subject specimen and a reference curve, $Pn(i)$. Herein, the deviation curve is denoted as $D(i)$. $D(i)$ for the subject specimen is expressed as the following equation (2):

$$D(i) = P(i) \text{ for subject specimen} - Pn(i) \quad (2).$$

The reference curve, $Pn(i)$, is prepared from $P(i)$ for a reference specimen (hereinafter, referred to as $P'(i)$). Applicable as the reference specimen is a blood specimen derived from an individual without blood coagulation disorder, for example, a blood specimen, preferably plasma, collected from a healthy individual, a pooled specimen thereof, or commercially available normal plasma (hereinafter, collectively referred to as a "normal specimen"). Preferably, the reference specimen is a specimen with a coagulation time (APTT) preferably from 24 to 39 seconds, more preferably from 24 to 30 seconds. Alternatively, $P(i)$ for a specimen with the shortest coagulation time among blood specimens derived from individuals without blood coagulation disorder, or the mean of $P(i)$ for blood specimens derived from individuals without blood coagulation disorder can be used as $P'(i)$.

$Pn(i)$ is a curve given by shifting $P'(i)$ to match a rising point thereof (time point or measuring point number corresponding thereto) with a specific point X. The specific point, X, is basically a point earlier than the rising point, preferably X = from 5 to 15 seconds, or measuring point number corresponding thereto. The rising point of P'(i) can be detected as a time point or measuring point number at which P(i) reaches from 1 to 5%, for example, 2% of a maximum value thereof. Let the difference between the detected rising point and the specific point, X, be k, Pn(i) is expressed as the following equation (3):

$$\mathrm{Pn(i) \ = \ P'(i+k) \quad (3).}$$

Accordingly, D(i) in an embodiment can be expressed as the following equation (2)':

$$\mathrm{D(i) \ = \ P(i) \ - \ P'(i+k) \quad (2)'.}$$

4. Evaluation of shape of deviation curve

In the present invention, a parameter reflecting the shape of the deviation curve D(i), given above for the subject specimen can be calculated. Examples of the parameter include weighted average time, weighted average height, and peak width.

In an embodiment, the parameter to be calculated is weighted average time and/or weighted average height in a specific calculation target area in the deviation curve D(i) for the subject specimen. The calculation target area is defined as an area satisfying $D(i) \geq D\max \times S(\%)$ in D(i), wherein Dmax denotes a maximum value of D(i), and $0 < S < 100$. Preferably, $1 \leq S \leq 99$, and more preferably $5 \leq S \leq 95$. Accordingly, the calculation target area and the weighted average time and weighted average height therein vary with S. Multiple weighted average times and weighted average heights can be calculated for different values of S. Herein, the weighted average time and weighted average height varying with S are denoted as gT(S) and gH(S), respectively.

gT(S) and gH(S) are expressed as the following equations (4) and (5), respectively:

$$\mathrm{gT(S)} \ = \ \frac{\sum_{i=t1}^{t2}(i \times D(i))}{\sum_{i=t1}^{t2} D(i)} \quad (4)$$

$$\mathrm{gH(S)} \ = \ \frac{\sum_{i=t1}^{t2}(i \times D(i))}{\sum_{i=t1}^{t2} i} \quad (5)$$

wherein t1 and t2 are respectively a minimum value and maximum value of i satisfying $D(i) \geq D\max \times S(\%)$, provided that if $D(i) < D\max \times S(\%)$, then i = 0 and D(i) = 0 are assigned.

In an embodiment, the parameter to be calculated is peak width in a specific calculation target area in the deviation curve D(i) for the subject specimen, and this indicates time length or the number of measuring points satisfying $D(i) \geq D\max \times S(\%)$ between the t1 and t2. Accordingly, the peak width varies with S. Multiple peak widths can be calculated for different values of S. Herein, the peak width varying with S is denoted as gB(S).

In an embodiment, the parameter to be calculated is the ratio between two values of gB(S) for different values of S, and this is specifically expressed as gB(S1) / gB(S2), and herein also referred to as RgB(S1/S2). Here, S1 and S2 are each defined the same as above-described S, provided that $S1 \neq S2$. Preferably, $|S1 - S2| \geq 20$; for example, $5 \leq S1 \leq 40$ and $60 \leq S2 \leq 90$, or $60 \leq S1 \leq 90$ and $5 \leq S2 \leq 40$.

In an embodiment, the parameter to be calculated is the ratio between gB(S) and gH(S), and this is specifically expressed as gH(S) / gB(S), wherein $5 < S < 90$ is preferably satisfied. Herein, gH(S) / gB(S) is also expressed as the sharpness, gAB(S).

5. Estimation of cause for coagulation disorder

The cause for coagulation disorder in the subject specimen can be estimated on the basis of the above-described parameter reflecting the shape of D(i).

In an embodiment of the present invention, whether a subject specimen without extension of coagulation time is normal or has received administration of DOACs (DOACs-positive) can be estimated.

In another embodiment of the present invention, for a subject specimen with extension of coagulation time, the cause for coagulation disorder which has resulted in the extension of coagulation time can be estimated. Examples of the cause for coagulation disorder to be estimated in the present invention include coagulation factor deficiency, lupus-anticoagulant positiveness, coagulation-factor-inhibitor positiveness, and heparin positiveness.

(1. Calculation of coagulation time)

Thus, in the present invention, coagulation time is calculated for the subject specimen before estimating the cause for coagulation disorder. The calculation of coagulation time can be performed with any method. Examples of the calculation method for coagulation time include, but are not limited to: a method of calculating coagulation time as a time point at which $P(i)$ has reached N% of a maximum value thereof; a method of calculating coagulation time as a time point at which the first derivative curve of P has reached N% of a maximum value thereof; a method of calculating coagulation time as a time point at which $P(i)$ has reached N% of $P(Te)$, wherein Te denotes a calculation starting point at which the ratio of a cumulative value of $P(i)$ in a minute time period has reached a specific value (JP-A-6-249855); a method of calculating coagulation time on the basis of the temporal variation of a cumulative value of $P(i)$ in a minute time period (see Japanese Patent Application No. 2019-237427); a method of calculating coagulation time on the basis of the weighted average time of the first derivative curve of P (see Japanese Patent Application No. 2020-039344); and a method of calculating coagulation time as a time point at which $P(i)$ has reached N% of $P(Te)$, wherein Te denotes a calculation starting point at which the first derivative curve of P has reached a specific value after reaching a maximum value (see Japanese Patent Application No. 2020-068877).

(2. Estimation of DOACs positiveness)

If the subject specimen does not exhibit extension of coagulation time, whether the specimen is normal or DOACs-positive is estimated on the basis of the parameter reflecting the shape of $D(i)$.

In an embodiment, the parameter is $gB(S)$. $gB(S)$ for DOACs-positive specimens tends to be greater than those for normal specimens. Accordingly, if $gB(S)$ for the subject specimen is equal to or greater than a preset threshold, the specimen can be estimated to be DOACs-positive. The threshold can be determined from $gB(S)$ given by a known DOACs-positive specimen group. For example, the threshold can be set as [mean - k × SD] of $gB(S)$ for a known DOACs-positive specimen group (preferably, k = from 0.5 to 3). If $gB(S)$ for the subject specimen is equal to or smaller than the preset threshold, on the other hand, the specimen can be estimated not to be DOACs-positive (or to be normal).

In another embodiment, the parameter is $gAB(S)$. $gAB(S)$ for DOACs-positive specimens tends to be smaller than those for normal specimens. Accordingly, if $gAB(S)$ for the subject specimen is equal to or smaller than a preset threshold, the specimen can be estimated to be DOACs-positive. The threshold can be determined from $gAB(S)$ given by a known DOACs-positive specimen group. For example, the threshold can be set as [mean + k × SD] of $gAB(S)$ for a known DOACs-positive specimen group (preferably, k = from 0.5 to 3). If $gAB(S)$ for the subject specimen is equal to or greater than the preset threshold, on the other hand, the specimen can be estimated not to be DOACs-positive (or to be normal).

(3. Estimation of cause for coagulation disorder)

If the subject specimen exhibits extension of coagulation time, the specimen has coagulation disorder which results in extension of coagulation time. The cause for coagulation disorder in the subject specimen is estimated on the basis of the parameter reflecting the shape of $D(i)$.

(3.1. Estimation of heparin positiveness)

In an embodiment, the parameter is $RgB(S1/S2)$. In the case of S1 < S2, $RgB(S1/S2)$ for heparin-positive specimens tends to be smaller than those for specimens with other-type coagulation disorder. Accordingly, if $RgB(S1/S2)$ for the subject specimen is equal to or smaller than a preset threshold, the specimen can be estimated to be heparin-positive (Hep). The threshold can be determined from $RgB(S1/S2)$ given by a known heparin-positive specimen group. For example, the threshold can be set as [mean + k × SD] of $RgB(S1/S2)$ for a known heparin-positive specimen group (preferably, k = from 0.5 to 3). In the case of S1 > S2, on the other hand, if $RgB(S1/S2)$ for the subject specimen is equal to or greater than the preset threshold, the specimen can be estimated to be heparin-positive (Hep), and the threshold in this case can be set as, for example, [mean - k × SD] of $RgB(S1/S2)$ for a known heparin-positive specimen group (preferably, k = from 0.5 to 3).

(3.2. Estimation of inhibitor positiveness)

In an embodiment, the parameter is $gAB(S)$. $gAB(S)$ for coagulation-factor-inhibitor-positive specimens tends to be smaller than those for specimens with other-type coagulation disorder. Accordingly, if $gAB(S)$ for the subject specimen is equal to or smaller than a preset threshold, the specimen can be estimated to be coagulation-factor-inhibitor-positive (Inh). The threshold can be determined from $gAB(S)$ given by a known coagulation-factor-inhibitor-positive specimen group. For example, the threshold can be set as [mean + k × SD] of $gAB(S)$ for a known coagulation-factor-inhibitor-positive specimen group (preferably, k = from 0.5 to 3).

(3.3. Estimation of lupus-anticoagulant positiveness and coagulation factor deficiency)

In an embodiment, the cause for coagulation disorder in a subject specimen which has been estimated not to be heparin-positive (Hep) or coagulation-factor-inhibitor-positive (Inh) in the above procedures is estimated. Specifically, whether the specimen is lupus-anticoagulant-positive (LA) or coagulation-factor-deficient (Def) is estimated.

For estimation of LA and Def, a mixed specimen of the subject specimen and a normal specimen is prepared, and a deviation curve D(i) for the mixed specimen is generated. Separately, D(i) for each of the subject specimen and the normal specimen is generated. gAB(S) is calculated from D(i) for each. The procedure to calculate gAB(S) for the subject specimen is as described above.

In preparing the mixed specimen, the subject specimen and the normal specimen are mixed at a specific ratio. Applicable as the normal specimen is one as described above, for example, a blood specimen, preferably plasma, collected from a healthy individual, a pooled specimen thereof, or commercially available normal plasma. The mixing ratio of the subject specimen and the normal specimen can be in the range of subject specimen:normal specimen = from 1:9 to 9:1, is preferably in the range from 4:6 to 6:4, and more preferably 5:5, in a volume ratio as the total is assumed as 10 volumes. Coagulation reaction in the mixed specimen is monitored to generate P(i) for the mixed specimen. Here, P(i) for the mixed specimen is also referred to as Pm(i). D(i) for the mixed specimen is calculated from this Pm(i). Here, D(i) for the mixed specimen is also referred to as Dm(i). Subsequently, gAB(S) is calculated from this Dm(i). Separately, coagulation reaction in the normal specimen is monitored to generate P(i) for the normal specimen. Here, P(i) for the normal specimen is also referred to as Ps(i). D(i) for the normal specimen is calculated from this Ps(i). Here, D(i) for the normal specimen is also referred to as Ds(i). Subsequently, gAB(S) is calculated from this Ds(i). Procedures for coagulation reaction monitoring for the mixed specimen and the normal specimen, generation of Pm(i) and Ps(i), and calculation of Dm(i) and Ds(i) accord with those described above for the subject specimen. Procedures to calculate gAB(S) from Dm(i) and from Ds(i) accord with that described above for gAB(S) for the subject specimen.

From gAB(S) for Dm(i) for the mixed specimen, D(i) for the subject specimen, and Ds(i) for the normal specimen, a parameter R(S), is calculated with the following equation (6):

$$R(S) = \{(gAB(S) \text{ for } Ds(i)) + (gAB(S) \text{ for } D(i))\} / 2 - (gAB(S) \text{ for } Dm(i)) \tag{6}$$

R(S) for LA specimens tends to be greater than those for Def specimens. Accordingly, if R(S) for the subject specimen is equal to or greater than a preset threshold, the specimen can be estimated to be LA; if R(S) is smaller than the preset threshold, on the other hand, the specimen can be estimated to be Def. The threshold can be determined from R(S) given by a known LA specimen group or Def specimen group. For example, the threshold can be set between a minimum vale of R(S) given by an LA specimen group and a maximum value of R(S) given by a Def specimen group. Alternatively, the threshold can be set as [mean - k × SD] of R(S) for an LA specimen group or [mean + k × SD] of R(S) for a Def specimen group (preferably, k = from 2 to 5).

Those skilled in the art should understand that in the above procedures to estimate the cause for coagulation disorder, "equal to or greater than a threshold" may be used in place of "greater than a threshold" and "equal to or smaller than a threshold" may be used in place of "smaller than a threshold", as long as different causes for coagulation disorder can be distinguished in the estimation.

The estimation result given through those procedures for the cause for coagulation disorder in the subject specimen can be outputted in any form. For example, the estimation result can be outputted in the form of electronic data, a print, or display on the screen. The estimation result contains information on whether the subject specimen is normal, or is DOACs-positive, coagulation-factor-deficient, lupus-anticoagulant-positive, coagulation-factor-inhibitor-positive, or heparin-positive. The coagulation time in the subject specimen, P(i) for the subject specimen, D(i) or a parameter reflecting the shape of D(i), or the value of the parameter R(S), may be outputted together with the estimation result.

6. Application to other coagulation reaction monitoring methods

Thus, the present invention has been described for the case of using a coagulation reaction curve generated on the basis of scattered light intensity as an example. However, those skilled in the art could apply the method of the present invention to a method with a coagulation reaction curve generated with any other coagulation reaction monitoring method (for example, a coagulation reaction monitoring method based on transmittance, absorbance, or viscosity). For example, the positive and negative signs in reverse-sigmoidal coagulation reaction curves as based on transmitted light intensity are reverse to those in coagulation reaction curves based on scattered light intensity as described above. It is obvious to those skilled in the art that in such a case, the signs of D(i) reverse in parameter calculation, for example, a maximum value of D(i), Dmax, is replaced with a minimum value thereof, Dmin, and a calculation target area is replaced with an area satisfying D(i) ≤ Dmin × S(%).

Examples

The following describes the present invention in more detail with reference to examples, but the present invention is not limited to those examples.

(Reference Example)

The following describes an example of the flow of procedures to estimate the cause for coagulation disorder.

(S1) Calculate a coagulation reaction curve, P(i), and coagulation time (APTT) for a subject specimen.
(S2) Calculate D(i) for the subject specimen. D(i) = P(i) - Pn(i).
(S3) Calculate a parameter reflecting the shape of D(i).
(S4) Compare the APTT with a reference value.

→ If extension of APTT is absent, proceed to (S5).
→ If extension of APTT is present, proceed to (S6).

(S5) Calculate parameter A [gB(S)] or parameter B [gAB(S)] for the subject specimen, and compare the parameter with a threshold.

→ If parameter A is greater than the threshold, proceed to (S11).
→ If parameter B is smaller than the threshold, proceed to (S11).
→ Otherwise, proceed to (S10).

(S6) Calculate a parameter [gAB(S)] for the subject specimen, and compare the parameter with a threshold.

→ If the parameter is smaller than the threshold, proceed to (S12).
→ Otherwise, proceed to (S7).

(S7) Calculate a parameter [RgB(S1/S2)] (S1 < S2) for the subject specimen, and compare the parameter with a threshold.

→ If the parameter is smaller than the threshold, proceed to (S13).
→ Otherwise, proceed to (S8).

(S8) Calculate D(i) for a mixed specimen and a normal specimen. Calculate the following parameter R(S), from gAB(S) for the mixed specimen, the subject specimen, and the normal specimen, and compare the parameter with a threshold.

R(S) = {(gAB(S) for normal specimen) + gAB(S) for subject specimen)} / 2 - (gAB(S) for mixed specimen)

→ If the parameter is greater than the threshold, proceed to (S14).
→ If the parameter is smaller than the threshold, proceed to (S15).

In the flow, the order of (S6) and (S7) is interchangeable. The calculation of D(i) for the mixed specimen and the normal specimen in (S8) may be carried out in advance (for example, in (S2)).

(S10) Output the result that the subject specimen is normal and the APTT.
(S11) Output the result that the subject specimen is DOACs-positive and the APTT.
(S12) Output the result that the subject specimen is Inh and the APTT.
(S13) Output the result that the subject specimen is Hep and the APTT.
(S14) Output the result that the subject specimen is LA and the APTT.
(S15) Output the result that the subject specimen is Def and the APTT.

Example 1)

1) Specimens

Reference specimen (RP): Pooled Normal Plasma from Precision BioLogic Inc.
Normal specimens (NP): plasma (N = 22) with APTT falling within the reference range (from 24 to 39 seconds) Abnormal specimens:

- LA-positive specimens (LA): Positive Lupus Anticoagulant Plasma from George King Biomedical Inc., and plasma from LA patients (N = 10 in total).
- Coagulation-factor-inhibitor-positive specimens (Inh): Factor VIII Deficient with Inhibitor from George King Biomedical Inc., and plasma from Inh patients (N = 9 in total).
- Coagulation-factor-deficient specimens (Def):
  factor V deficient plasma, factor VIII deficient plasma (HA), factor IX deficient plasma (HB), factor X deficient plasma, factor XI deficient plasma (Factor VIII Deficient and Factor IX Deficient from George King Biomedical Inc., and plasma from patients; N = 19 in total).
- DOACs-positive specimens (DOACs): plasma from patients with administration of DOACs (N = 5, four specimens of them exhibited APTT within the reference range).
- Heparin-positive specimens (Hep): plasma from Hep patients, and RP with unfractionated heparin added thereto (N = 18 in total).

Mixed specimens: 1:1 mixed plasma of the LA and NP (N = 8), 1:1 mixed plasma of the Def and NP (N = 6, five of them were mixed plasma of HA and NP, the residual one was mixed plasma of HB and NP).

2) Coagulation reaction monitoring

Reagents used for APTT measurement were Coagpia APTT-N (manufactured by SEKISUI MEDICAL CO., LTD.) as a first reagent and Coagpia APTT-N calcium chloride solution (25 mM calcium chloride solution, manufactured by SEKISUI MEDICAL CO., LTD.) as a second reagent. Coagulation reaction monitoring was carried out by using the automatic blood coagulation analyzer CP3000 (manufactured by SEKISUI MEDICAL CO., LTD.). After warming 50 $\mu$L of a specimen in a cuvette at 37°C for 45 seconds, 50 $\mu$L of the first reagent at approximately 37°C was added, and 171 seconds after that 50 $\mu$L of the second reagent was further added to initiate coagulation reaction. The reaction was caused at 37°C. In monitoring of the coagulation reaction, the cuvette was irradiated with light having a wavelength of 660 nm from an LED light source, and the scattered light intensity of 90-degree side scatter was measured at intervals of 0.1 seconds. The monitoring period was 360 seconds.

3) Generation of coagulation reaction curve, P(i)

The photometric data, C(i), for each specimen was subjected to smoothing including noise removal, and then to zero adjustment to adjust the scattered light intensity at the time point of starting photometry to 0. C(i) after the adjustment was relativized with the above equation (1) to set the maximum value to 100, giving a coagulation reaction curve, P(i).

4) Generation of reference curve, Pn(i)

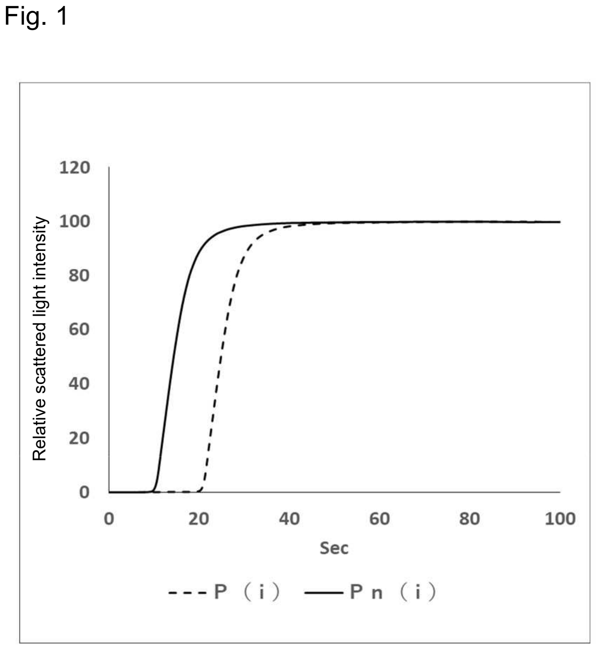

P(i) from RP was shifted to match the rising time point with 10 seconds, giving Pn(i). The rising time point of P(i) was detected as a time point at which P(i) reached 2% of a maximum value thereof (maximum scattered light intensity). Fig. 1 shows P(i) for RP and Pn(i).

5) Generation of deviation curve D(i)

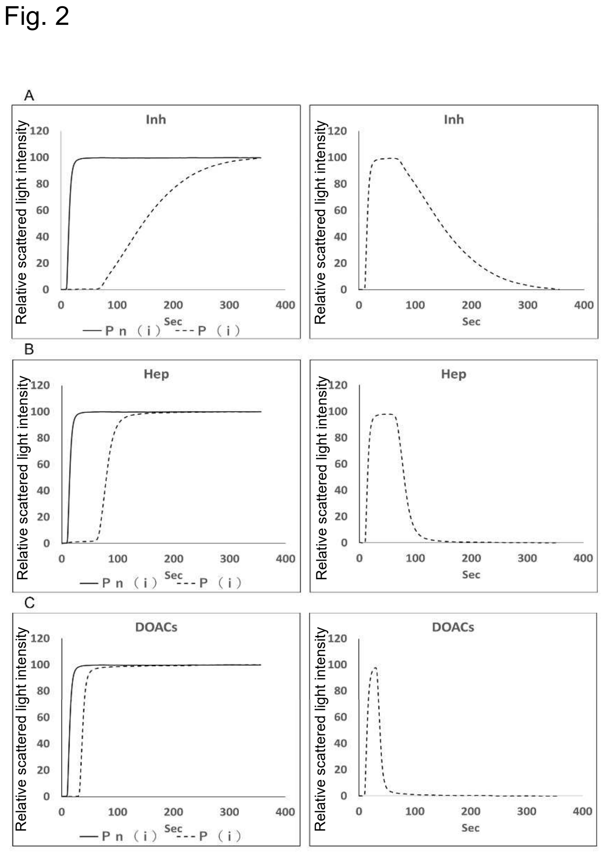

From P(i) for each specimen and Pn(i), D(i) was generated with the above equation (2). Fig. 2 shows P(i) and D(i) generated from Inh (A), Hep (B), and DOACs (C). In each case, the left graph shows P(i) for the specimen together with Pn(i), and the right graph shows D(i). P(i) for Inh exhibited delayed rising and slow elevation, and thus D(i) showed a wide peak and slow lowering (Fig. 2A). P(i) for Hep exhibited delayed rising but rapid elevation, and thus D(i) showed a wide peak but quick elevation and lowering (Fig. 2B). P(i) for DOACs exhibited early rising and rapid elevation, and thus D(i) showed a sharp peak (Fig. 2C).

6) Calculation of parameters

gT(S), gH(S), and gB(S) were calculated from D(i) for each specimen. S was varied in the range from 5 to 90, and gT(S) and gH(S) were calculated for each value of S with the above equations (4) and (5). Between t1 and t2 in the equation (4) for each value of S, time length or the number of measuring points satisfying D(i) ≥ Dmax × S(%) was determined as gB(S). Furthermore, gAB(S) was calculated. From gAB(S) for a mixed specimen, an abnormal specimen contained therein, and RP, a parameter R(S), was calculated with the above equation (6). Hereinafter, gT(S), gH(S), gB(S), gAB(S), and R(S) may be written as gTS, gHS, gBS, gABS, and RS, respectively, with the parentheses omitted.

7) Estimation of DOACs-positive specimens

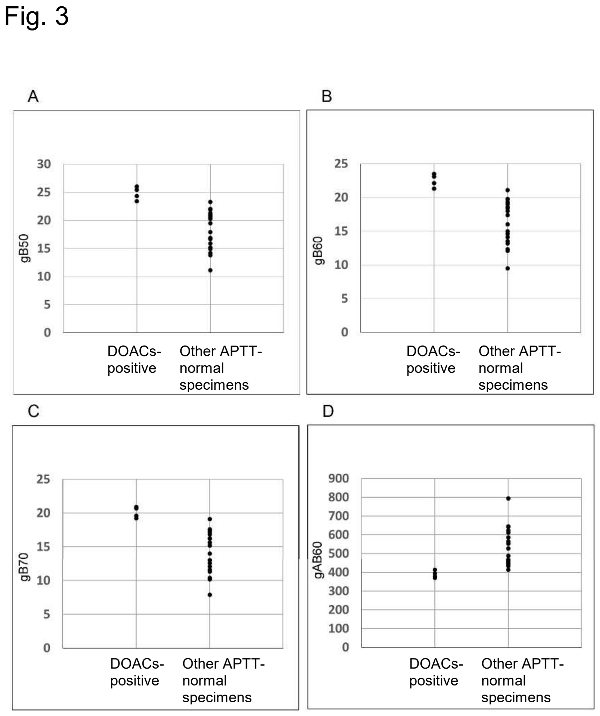

Of 83 specimens, 26 specimens were normal for APTT (within the reference range, no extension) (APTT ≤ 39), and 22 specimens thereof were NP and the residual four specimens were DOACs. Figs. 3A to 3D show gB50, gB60, gB70, and gAB60 for those specimens normal for APTT. gB50 to gB70 for DOACs tended to be greater than those for other APTT-normal specimens (that is, NP), thus showing different distribution. gAB60 for DOACs tended to be smaller than that for other APTT-normal specimens (NP), thus showing different distribution. It was demonstrated that normal specimens and DOACs-positive specimens can be distinguished from each other on the basis of gB(S) and gAB(S).

8) Estimation of coagulation-factor-inhibitor-positive specimens

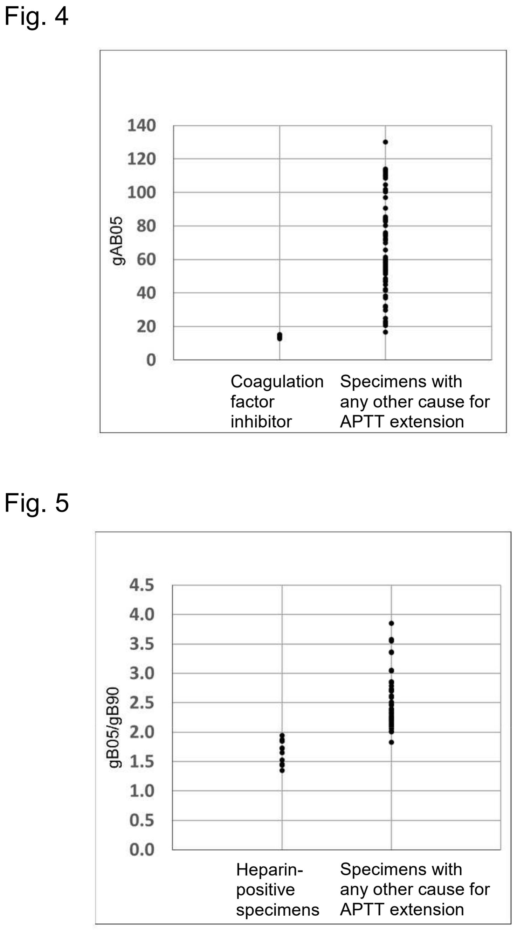

Fifty-seven specimens exhibited extension of APTT (APTT > 39), and nine specimens thereof were Inh. Fig. 4 shows gAB05 for Inh specimens and specimens with any other cause for APTT extension. gAB05 for Inh tended to be smaller than those for other specimens. It was demonstrated that coagulation-factor-inhibitor-positive specimens can be distinguished from abnormal specimens with APTT extension on the basis of gAB(S).

9) Estimation of heparin-positive specimens

For 48 specimens given by eliminating the nine Inh specimens from the 57 specimens used in 8), RgB(05/90) (gB05 / gB90) was examined. Of the 48 specimens, 18 specimens were Hep. Fig. 5 shows gB05 / gB90 for Hep specimens and specimens with any other cause for APTT extension. gB05 / gB90 for Hep tended to be smaller than those for other specimens. Though, one factor-X-deficient specimen showed overlapping with the distribution for Hep. It was demonstrated that heparin-positive specimens can be distinguished from abnormal specimens with APTT extension on the basis of the ratio of gB(S) for different values of S, RgB(S1/S2) (gB(S1) / gB(S2)).

10) Estimation of LA and Def

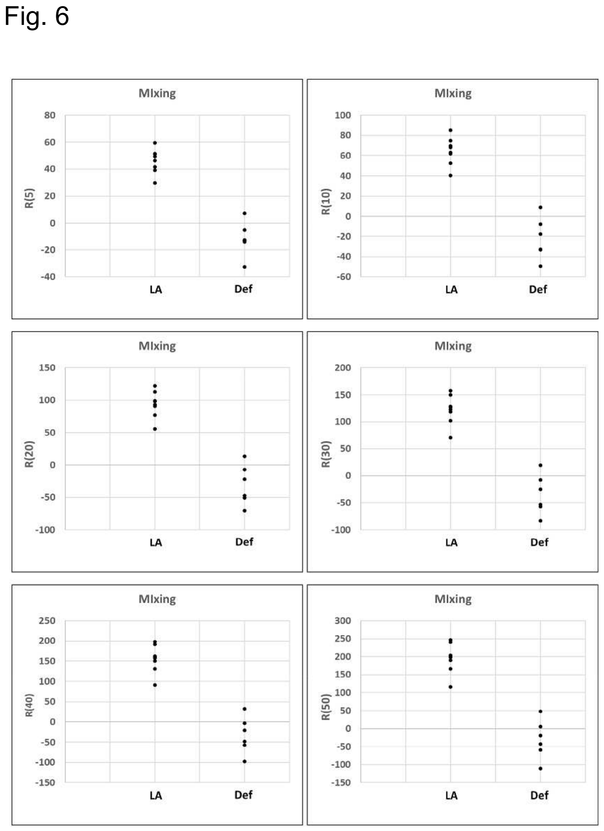

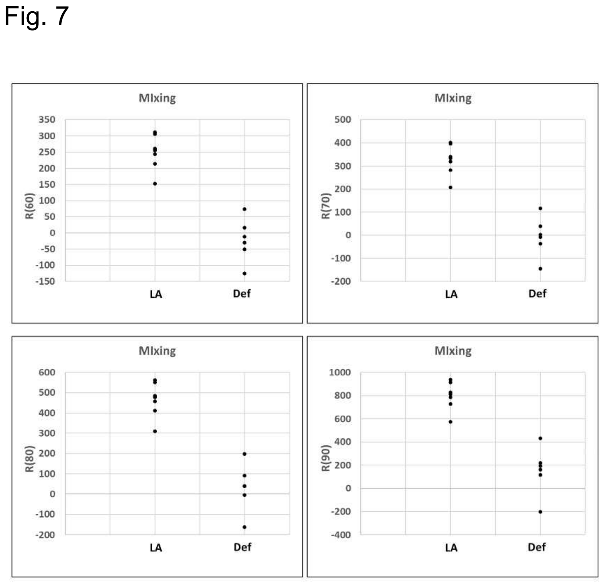

For 14 mixed specimens and abnormal specimens used for preparation thereof (eight LA specimens, six Def specimens), and NP, gAB(S) was calculated for values of S of 5, 10, 20, 30, 40, 50, 60, 70, 80, and 90, and then R(S) was examined. Figs. 6 and 7 show R(S) (S = 5, 10, 20, 30, 40, 50, 60, 70, 80, and 90) for LA and Def. R(S) for LA tended to be greater than those for Def. It was demonstrated that LA-positive specimens and coagulation-factor-deficient specimens can be distinguished from each other on the basis of R(S).

**Claims**

1. A method for estimating a cause for coagulation disorder in a blood specimen, the method comprising:

   calculating D(i), which is a deviation curve for a subject blood specimen;
   calculating a parameter reflecting a shape of D(i); and
   estimating a cause for coagulation disorder in the subject blood specimen on a basis of the parameter,

   wherein

$$D(i) = P(i) - Pn(i),$$

   wherein

   i denotes measuring point number, or time,

P(i) is a curve given by relativizing measurement data for coagulation reaction in the subject blood specimen to give a minimum value of 0 and a maximum value of A,

Pn(i) is a curve given by shifting P'(i) to match a rising point thereof with a specific point X, wherein the P'(i) is a curve given by relativizing measurement data for coagulation reaction in a reference specimen to give a minimum value of 0 and a maximum value of A,

$$A > 0,$$

and

X is from 5 to 15 seconds, or measuring point number corresponding thereto.

2. The method according to claim 1, wherein the parameter reflecting the shape of D(i) is at least one selected from the group consisting of gT(S), gH(S), gB(S), gAB(S), and RgB(S1/S2), wherein

gT(S) and gH(S) are expressed as the following equations:

$$gT(S) = \frac{\sum_{i=t1}^{t2}(i \times D(i))}{\sum_{i=t1}^{t2} D(i)}$$

$$gH(S) = \frac{\sum_{i=t1}^{t2}(i \times D(i))}{\sum_{i=t1}^{t2} i}$$

wherein

t1 and t2 are respectively a minimum value and maximum value of i satisfying $D(i) \geq Dmax \times S(\%)$, wherein Dmax denotes a maximum value of D(i), and 0 < S < 100,

provided that if $D(i) < Dmax \times S(\%)$, then i = 0 and D(i) = 0 are assigned,

gB(S) represents time length or the number of measuring points satisfying $D(i) \geq Dmax \times S(\%)$ between t1 and t2,

$$gAB(S) = gH(S) / gB(S),$$

and

$$RgB(S1/S2) = gB(S1) / gB(S2),$$

wherein

0 < S1 < 100 and 0 < S2 < 100, provided that S1 ≠ S2.

3. The method according to claim 2, comprising calculating at least one selected from the group consisting of gB(S) and gAB(S) if extension of coagulation time is absent in the subject blood specimen.

4. The method according to claim 3, comprising estimating the subject blood specimen to be DOACs-positive if gB(S) is equal to or greater than a threshold or gAB(S) is equal to or smaller than a threshold.

5. The method according to claim 2, comprising calculating at least one selected from the group consisting of gAB(S) and RgB(S1/S2) if extension of coagulation time is present in the subject blood specimen.

6. The method according to claim 5, comprising estimating the subject blood specimen to be coagulation-factor-inhibitor-positive if gAB(S) is equal to or smaller than a threshold.

7. The method according to claim 5, comprising estimating the subject blood specimen to be heparin-positive if S1 < S2, and RgB(S1/S2) is equal to or smaller than a threshold.

8. The method according to claim 5, comprising:

estimating the subject blood specimen to be coagulation-factor-inhibitor-positive if gAB(S) is equal to or smaller than a threshold;

estimating the subject blood specimen to be heparin-positive if S1 < S2, and RgB(S1/S2) is equal to or smaller than a threshold; and

estimating whether a subject blood specimen estimated not to be coagulation-factor-inhibitor-positive or heparin-positive is lupus-anticoagulant-positive or coagulation-factor-deficient.

9. The method according to claim 8, comprising:

preparing a mixed specimen of the subject blood specimen estimated not to be coagulation-factor-inhibitor-positive or heparin-positive and a normal specimen;

calculating Dm(i), which is a deviation curve for the mixed specimen, and Ds(i), which is a deviation curve for the normal specimen;

calculating gAB(S) for each of Dm(i) and Ds(i); and

calculating a parameter R(S), wherein

$$Dm(i) = Pm(i) - Pn(i),$$

$$Ds(i) = Ps(i) - Pn(i),$$

wherein

Pm(i) is a curve given by relativizing measurement data for coagulation reaction in the mixed specimen to give a minimum value of 0 and a maximum value of A,

Ps(i) is a curve given by relativizing measurement data for coagulation reaction in the normal specimen to give a minimum value of 0 and a maximum value of A, and

Pn(i) and A are as defined above,

gAB(S) for Ds(i) is expressed as the following equation:

gAB(S) for Ds(i) = [gH(S) for Ds(i)] / [gB(S) for Ds(i)]

wherein

$$gH(S) \ for \ Ds(i) = \frac{\sum_{i=t1}^{t2}(i \times Ds(i))}{\sum_{i=t1}^{t2} i} \qquad (I)$$

wherein

t1 and t2 are respectively a minimum value and maximum value of i satisfying Ds(i) ≥ [maximum value of Ds(i) × S(%)],

$$0 < S < 100,$$

provided that if Ds(i) < [maximum value of Ds(i) × S(%)], then i = 0 and Ds(i) = 0, and

gB(S) for Ds(i) represents time length or the number of measuring points satisfying Ds(i) ≥ [maximum value of Ds(i) × S(%)] between t1 and t2,

gAB(S) for Dm(i) is expressed as the following equation:

gAB(S) for Dm(i) = [gH(S) for Dm(i)] / [gB(S) for Dm(i)]

wherein

$$gH(S) \ for \ Dm(i) = \frac{\sum_{i=t1}^{t2}(i \times Dm(i))}{\sum_{i=t1}^{t2} i} \qquad (II)$$

wherein

t1 and t2 are respectively a minimum value and maximum value of i satisfying Dm(i) ≥ [maximum value of Dm(i) × S(%)],

$$0 < S < 100,$$

provided that if Dm(i) < [maximum value of Dm(i) $\times$ S(%)], then i = 0 and Dm(i) = 0 are assigned, and gB(S) for Dm(i) represents time length or the number of measuring points satisfying Dm(i) $\geq$ [maximum value of Dm(i) $\times$ S(%)] between t1 and t2, and R(S) = {(gAB(S) for Ds(i)) + (gAB(S) for D(i))} / 2 - (gAB(S) for Dm(i)).

10. The method according to claim 9, comprising estimating the subject blood specimen to be lupus-anticoagulant-positive if R(S) is equal to or greater than a threshold, or estimating the subject blood specimen to be coagulation-factor-deficient if R(S) is equal to or smaller than a threshold.

11. The method according to claim 1, further comprising calculating coagulation time for the subject blood specimen.

Fig. 1

# Fig. 2

Fig. 3

Fig. 4

Fig. 5

Fig. 6

# Fig. 7

## INTERNATIONAL SEARCH REPORT

| International application No. |
| --- |
| **PCT/JP2024/019652** |

**A. CLASSIFICATION OF SUBJECT MATTER**

*G01N 33/86*(2006.01)i
FI: G01N33/86

According to International Patent Classification (IPC) or to both national classification and IPC

**B. FIELDS SEARCHED**

Minimum documentation searched (classification system followed by classification symbols)

G01N33/86

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2022/054819 A1 (SEKISUI MEDICAL CO., LTD.) 17 March 2022 (2022-03-17) entire text, all drawings | 1-11 |
| A | WO 2022/186381 A1 (SEKISUI MEDICAL CO., LTD.) 09 September 2022 (2022-09-09) entire text, all drawings | 1-11 |
| A | WO 2020/101025 A1 (SEKISUI MEDICAL CO., LTD.) 22 May 2020 (2020-05-22) entire text, all drawings | 1-11 |
| A | WO 2020/158948 A1 (SEKISUI MEDICAL CO., LTD.) 06 August 2020 (2020-08-06) entire text, all drawings | 1-11 |
| A | WO 2020/218425 A1 (SEKISUI MEDICAL CO., LTD.) 29 October 2020 (2020-10-29) entire text, all drawings | 1-11 |
| A | WO 2021/177452 A1 (SEKISUI MEDICAL CO., LTD.) 10 September 2021 (2021-09-10) entire text, all drawings | 1-11 |

☐ Further documents are listed in the continuation of Box C.    ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **31 July 2024** | **13 August 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/019652**

| Patent document cited in search report | | | Publication date (day/month/year) | Patent family member(s) | | | Publication date (day/month/year) |
|---|---|---|---|---|---|---|---|
| WO | 2022/054819 | A1 | 17 March 2022 | US | 2024/0027476 | A1 | |
| | | | | entire text, all drawings | | | |
| | | | | EP | 4212880 | A1 | |
| | | | | CN | 116057383 | A | |
| WO | 2022/186381 | A1 | 09 September 2022 | US | 2024/0077504 | A1 | |
| | | | | entire text, all drawings | | | |
| | | | | EP | 4303586 | A1 | |
| | | | | CN | 116917739 | A1 | |
| WO | 2020/101025 | A1 | 22 May 2020 | US | 2021/0333295 | A1 | |
| | | | | entire text, all drawings | | | |
| | | | | EP | 3882628 | A1 | |
| | | | | CN | 113039436 | A | |
| WO | 2020/158948 | A1 | 06 August 2020 | US | 2022/0146537 | A1 | |
| | | | | entire text, all drawings | | | |
| | | | | EP | 3919913 | A1 | |
| | | | | CN | 113366319 | A | |
| WO | 2020/218425 | A1 | 29 October 2020 | US | 2022/0326262 | A1 | |
| | | | | entire text, all drawings | | | |
| | | | | EP | 3961211 | A1 | |
| | | | | CN | 113785197 | A | |
| WO | 2021/177452 | A1 | 10 September 2021 | US | 2023/0152335 | A1 | |
| | | | | entire text, all drawings | | | |
| | | | | EP | 4116714 | A1 | |
| | | | | CN | 115244402 | A | |

Form PCT/ISA/210 (patent family annex) (July 2022)

**EP 4 722 724 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020101025 A
- WO 2020158948 A
- WO 2020218425 A
- WO 2021177452 A
- WO 2022054819 A
- WO 2022186381 A
- JP 6249855 A
- JP 2019237427 A
- JP 2020039344 A
- JP 2020068877 A